# EUROPEAN PATENT APPLICATION

(11) **EP 3 567 599 A1**
(43) Date of publication of application: **13.11.2019**
(21) Application number: 19181384.9
(22) Date of filing: 24.06.2016
(51) Int. Cl.: G16H 30/40, G16H 30/20, G16H 70/20, G16H 30/00, G06F 17/24, G06T 7/00, G16H 40/63, G16H 80/00

(54) **SYSTEM AND METHODS FOR IMPLEMENTING WOUND THERAPY PROTOCOLS**

(30) Priority: 26.06.2015 US 201562185320 P
(62) Divisional of application: 16745890.0
(71) Applicant: KCI Licensing, Inc., San Antonio, TX 78265 (US)
(72) Inventor: ALLEN, Diwi, L., San Antonio, TX Texas 78255 (US); GALAN, Gustavo, Boerne, TX Texas 78006 (US); GROTHUES, Hannah, I., Pleasanton, TX Texas 78064 (US); HILL, Robert, New Braunfels, TX Texas 78132 (US); COLLINS, Barbara, Anne, San Antonio, TX Texas 78258 (US); EDLUND, Chester,R., San Antonio, TX Texas 78265 (US); KIESWETTER, Kristine, M., San Antonio, TX Texas 78210 (US); DASGUPTA, Somesh, San Antonio, TX Texas 78257 (US); HAGGERTY, Joshua, San Antonio, TX Texas 78248 (US)
(74) Representative: Simmons & Simmons

(57) **Abstract**

Systems, methods, and apparatuses for treating a tissue site are described. In some embodiments, the method may include receiving input information related to a tissue site of a patient, processing the input information in a processor to generate options for a user to select a desired action, and executing the desired action to provide output information related to the tissue site. The method may further include communicating the output information to a user via a communications network.

## Description

### TECHNICAL FIELD

This disclosure relates generally to providing wound treatment in relation to systems for treating tissue sites and monitoring the usage of such systems, and the acquisition of such systems. More particularly, but not by way of limitation, the disclosure relates to an application for supporting clinicians in the wound treatment arena.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds with reduced pressure may be commonly referred to as "negative-pressure therapy," but is also known by other names, including "negative-pressure wound therapy," "reduced-pressure wound therapy," "vacuum therapy," and "vacuum-assisted closure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

Advanced wound care therapies (dressings, collagen, silver impregnated dressings, etc.) cover all wound healing phases and aide in the successful treatment of difficult and complex wounds. These therapies offer a variety of benefits such as; antimicrobial, absorbent, collagen enhancing, barrier, primary intention, moist dressing. Choosing the right dressing to suit the conditions of a patient's wound is vital for optimum healing and quality of life.

Skin grafts have been used to achieve successful wound healing when primary wound closure is not a feasible repair option. Epidermal skin grafts offer an alternative to traditional autografts and use only a minimal amount of autologous tissue from the donor site.

One of the roles of a health care professional is to examine the products available and reach a decision regarding which of the different types of dressings will be used in their trust. That decision will be made following evaluation of the products to ensure they perform well. It is also crucial to ensure that the dressings meet the needs of patients and do not cause any unnecessary discomfort on application, while the dressing is being worn, or on removal.

While the clinical benefits of reduced-pressure therapy are widely known, the cost and complexity of reduced-pressure therapy as well as other advanced wound care therapies and skin grafts, can be a limiting factor in their applications. In particular, when presented with a patient potentially in need of reduced-pressure wound therapy or other form of advanced wound care therapy, many clinicians may face difficulty in knowing how to properly assess the wound for determining the appropriate form of therapy. Currently, there is a need for providing a better way to assist clinicians with making such wound assessments as well as providing a streamlined process for the clinicians to make the appropriate therapy selections. Furthermore, current processes for ordering V.A.C.® Therapy is perceived by customers to be cumbersome and lengthy. For example, a valid prescription is required and the most predominant current method of sending a valid prescription is via fax, which often results in lost faxes, and thus lost orders for therapy equipment.

### BRIEF SUMMARY

According to an illustrative embodiment, a processor-implemented method may include receiving input information related to a tissue site of a patient, providing the input information to a processor for evaluation, processing the input information in the processor to generate options for a user to select a desired action, and executing the desired action on the processor to provide output information related to the tissue site. The method may further include providing the output information from the processor to a user via a communications network.

According to another illustrative embodiment, a computer-implemented method for treating a tissue site may include receiving input information related to a request for a consultation from a first mobile device, processing the input information in a processor to identify a selection of consult matches, and communicating output information through a communications network to a second mobile device. The method may further include calculating geographical data for a group of consult candidates as well as receiving feedback information from the second mobile device related to the request for a consultation.

According to yet another illustrative embodiment, a system for treating a tissue site may include a computer processing system comprising a processor adapted to receive input information related to a tissue site and to generate output information, a mobile device adapted to collect input information related to the tissue site and display output information relating to the tissue site, and a network adapted to allow communications between the computer processing system and the mobile device. A database configured for receiving and storing information related to the tissue site may also be included. The information may include one or more parameters related to healing of the tissue site and/or descriptors related to various therapy products and protocols.

Other objects, features, and advantages of the embodiments described herein will become apparent with reference to the drawings and detailed description that follow.

### BRIEF DESCRIPTION OF THE DRAWINGS

FIGURE 1A is an illustration of a therapy network in accordance with an exemplary embodiment;
FIGURE 1B illustrates a schematic diagram of a data flow map in accordance with an exemplary embodiment of the therapy network of FIGURE 1A;
FIGURE 2A illustrates screen shots of a graphical user interface (GUI) of a mobile wound management software for operation on an electronic device, according to an exemplary embodiment;
FIGURE 2B illustrates screen shots of a graphical user interface (GUI) of a mobile wound management software for operation on an electronic device, according to an exemplary embodiment;
FIGURE 2C illustrates screen shots of a graphical user interface (GUI) of a mobile wound management software for operation on an electronic device, according to an exemplary embodiment.
FIGURE 3A is a perspective view illustrating additional details that may be associated with some example embodiments of the therapy network of Figure 1;
FIGURE 3B illustrates a screen shot of a graphical user interface (GUI) of a mobile wound management software for operation on an electronic device that may be associated with some example embodiments of the therapy network according to Figure 1;
FIGURE 4 illustrates a screen shot of a graphical user interface (GUI) of a wound management software for operation on an electronic device, according to an exemplary embodiment;
FIGURE 5 illustrates a functional diagram of a wound management software according to an exemplary embodiment that may be associated with some example embodiments of the therapy network according to FIGURE 1; and
FIGURE 6 is a flow diagram of an exemplary process for managing wound treatment, which may be associated with some example embodiments of the therapy network of Figure 1.

### DETAILED DESCRIPTION OF ILLUSTRATIVE EMBODIMENTS

In the following detailed description of non-limiting, illustrative embodiments, reference is made to the accompanying drawings that form a part hereof. Other embodiments may be utilized, and logical, structural, mechanical, electrical, and chemical changes may be made without departing from the scope of the appended claims. To avoid detail not necessary to enable those skilled in the art to practice the embodiments described herein, the description may omit certain information known to those skilled in the art. The following detailed description is non-limiting, and the scope of the illustrative embodiments are defined by the appended claims. As used herein, unless otherwise indicated, "or" does not require mutual exclusivity.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

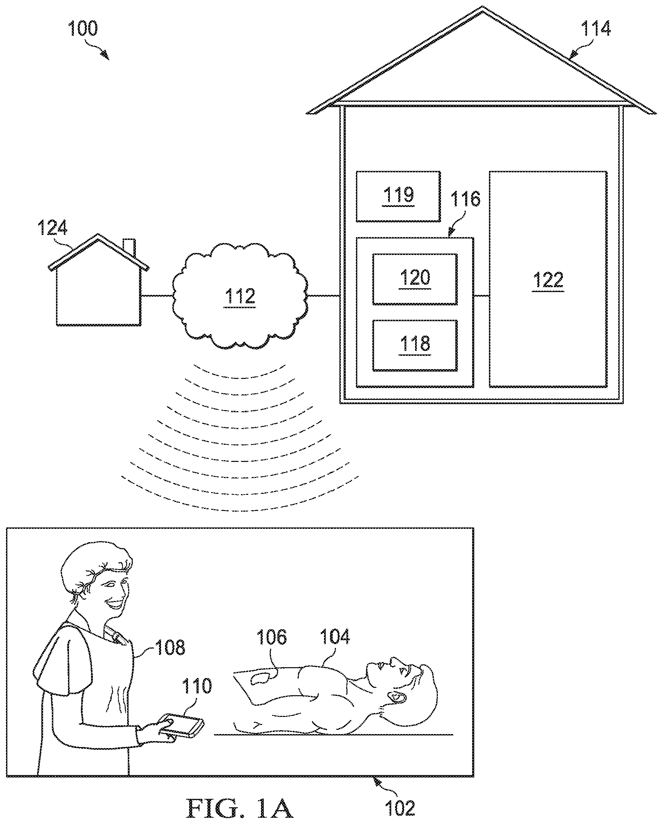

Figure 1A is a schematic diagram of an example embodiment of a therapy network 100 that can support a wound management application in accordance with this specification. The therapy network 100 may include a clinical setting 102, which may include an environment where a patient 104 with a tissue site 106 may be evaluated and/or treated by a clinician 108. The clinician 108 may use a mobile device 110, in conjunction with the wound management application, to enter, document, or record information related to the tissue site 106.

The term "tissue site" in this context broadly refers to a wound or defect located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be used in certain tissue areas to grow additional tissue that may be harvested and transplanted to another tissue location.

A mobile device for the purposes of this application may be any combination of a computer or microprocessor. The computer or microprocessor may be programmed to implement one or more software algorithms for achieving the functionality described in the specification and corresponding figures. The mobile device, such as mobile device 110, may also include a communication device, and may be a smartphone, a tablet computer, or other device that is capable of storing a software application programmed for a specific operating system (e.g., iOS, Android, and Windows). The mobile device 110 may also include an electronic display, such as a graphical user interface (GUI), for providing visual images to a user, such as a clinician or patient. The mobile device 110 may be configured to communicate with one or more networks 112 of the therapy network 100. In one preferred embodiment, the mobile device 110 may include a cellular modem and may be configured to communicate with the network(s) 112 through a cellular connection. In other embodiments, the mobile device 110 may include a Bluetooth® radio or other wireless radio technology for communicating with the network(s) 112. The mobile device 110 may be configured to transmit data related to the tissue site 106 of the patient 104.

The therapy network 100 may also include a therapy support center 114 that may be in communication with the mobile device 110 through network(s) 112. For example, the mobile device 110 may be configured to transmit data through network(s) 112 to the therapy support center 114. The therapy support center 114 may support a wound management application database 119. The therapy support center 114 may include a sales and clinical support center 116. The sales and clinical support center 116 may include a sales representative center 118 as well as a clinical team center 120. The sales representative center 118 may function as a centralized center for clinicians to contact regarding questions they may have related to therapy options for their patients. Currently, a Centralized Clinical Team (CCT) may provide clinical guidance to clinicians in a post-acute marketplace while simultaneously collecting wound assessments that may be required by third-party payers. In some cases, these required wound assessments are utilized for re-authorization of reduced-pressure therapy usage or other advanced wound care therapy, which thus are critical for providing continuity of care for patients. Each CCT member may manage a certain number of post-acute providers, such as clinicians, based on geographic location. The CCT may also collaborate with regional representatives in order to set up training opportunities for clinical providers.

In some embodiments, the wound management application may function to automatically route consultation requests originating from clinicians through the sales representative center 118 to the appropriate regional sales representatives and their corresponding assigned team members. In some embodiments, texting capabilities may be enabled between clinicians and the sales representatives through the wound management application and the network(s) 112 in a secure manner. The clinical team center 120 of the sales and clinical support center 116 may be configured to receive and triage alert notifications communicated through the wound management application from mobile devices in the therapy network 100, such as mobile device 110. The therapy support center 114 may also include a products disbursement center 122.

The products disbursement center 122 may serve to fulfill product orders that may be communicated through the wound management application and network(s) 112. For example, in some embodiments, the clinician may communicate with his or her designated sales representative either through the sales representative center 118, or directly, to order appropriate wound dressing products. The wound dressing product orders may then be communicated to the products disbursement center 122, which may complete fulfillment of the orders to either the clinician, the sales representative, or in some cases directly to a patient. In other embodiments, the clinician may directly communicate with the products disbursement center 122 through the wound management application using mobile device 110 over network(s) 112 in order to procure wound dressing products. In further embodiments, the products disbursement center 112 may fulfill routine refill or replacement orders according to a pre-arranged schedule and/or timeline.

The therapy network 100 may also include other entities that may communicate with clinical settings, mobile devices, and regional sales centers through network(s) 112. For example, the therapy network 100 may include a third party 124. For example, the third party 124 may be an insurer. In one embodiment, the third party 124 may communicate through the wound management application over network(s) 112 with the mobile device 110 in order to acquire wound description information. Such wound description information may be used for outcomes management purposes, for example to aid in expediting the collection of information that payers, such as insurers, require for reimbursement authorization. For example, a clinician may directly contact a clinical representative in a HIPAA-compliant format using standardized wound descriptors. In another embodiment, the third party 124 may communicate through network(s) 112 with the therapy support center 114 in order to acquire wound treatment information as well as prescription therapy information.

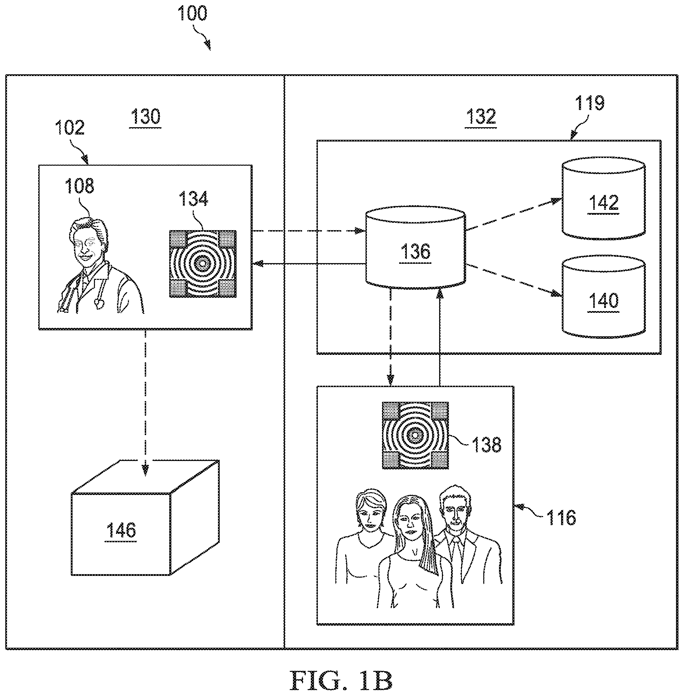

Figure 1B is a schematic diagram of an example embodiment of a data flow map corresponding to the therapy network 100 of Figure 1A. The data flow map of Figure 1B depicts both an external data environment 130 as well as an internal data environment 132. A clinician, such as the clinician 108 evaluating a patient in clinical setting 102, may interact with the wound management application through the external application 134, which may be accessed by the clinician 108 through a graphical user interface (GUI) of a mobile device, such as mobile device 110. The external application 134 may present the clinician 108 with various options for entering or referencing data related to specific wounds and possible therapy regimens. For example, data related to consult requests, specific prescription orders for therapy, as well as wound healing progression data may be accessed or entered by the clinician 108. The external application 134 may then communicate through one or more networks, such as network(s) 112, with a secure server hosting the wound management application, such as the iOn Healing Database 136 of the wound management application database 119. For example, consult request data, therapy prescription order data, and wound healing progression data may be communicated from the external application 134 of the external data environment 130 to the iOn Healing Database 136 of the internal data environment 132. Depending on the type of data, the iOn Healing Database 136 may then further route the received data within the internal data environment 132. For example, the iOn Healing Database 136 may route consult requests to users interacting with the wound management application through an internal application 138. In some embodiments of the therapy network 100, the users may be associated with the sales and clinical support center 116. The iOn Healing Database 136 may also route prescription therapy orders to the negative-pressure wound therapy (NPWT) Orders Database 142. From the NPWT Orders Database 142, orders for specific therapeutic products associated with the therapy prescribed may be generated and/or routed to the products disbursement center 122 of the therapy support center 114. Additionally, the iOn Healing Database 136 may communicate data regarding one or more specific wounds of a patient, such as wound healing progression data, to the NPWT Outcomes Database 140. In some embodiments, team members of the sales and clinical support center 116 may receive the wound healing progression data and move the data to a third party, such as an insurance payer for processing. In some embodiments, the data related to one or more specific wounds of a patient, such as wound healing progression data, may also be communicated in either its original or processed form from the iOn Healing Database 136 back to the external data environment 130. For example, in some embodiments, wound healing progression data may be processed and reported back to the clinician 108 through the external application 134 as well as in a format compatible for use as patient medical records to a facility, such as clinician facility 146.

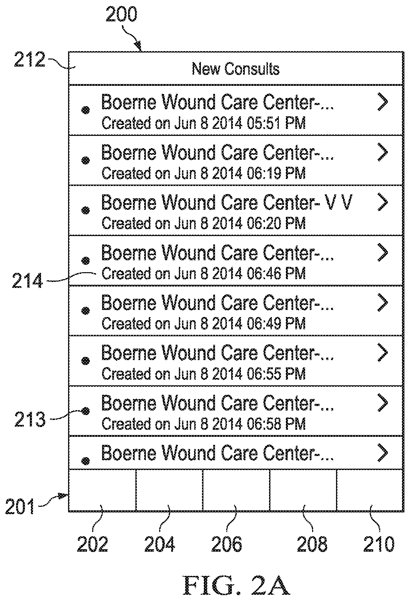

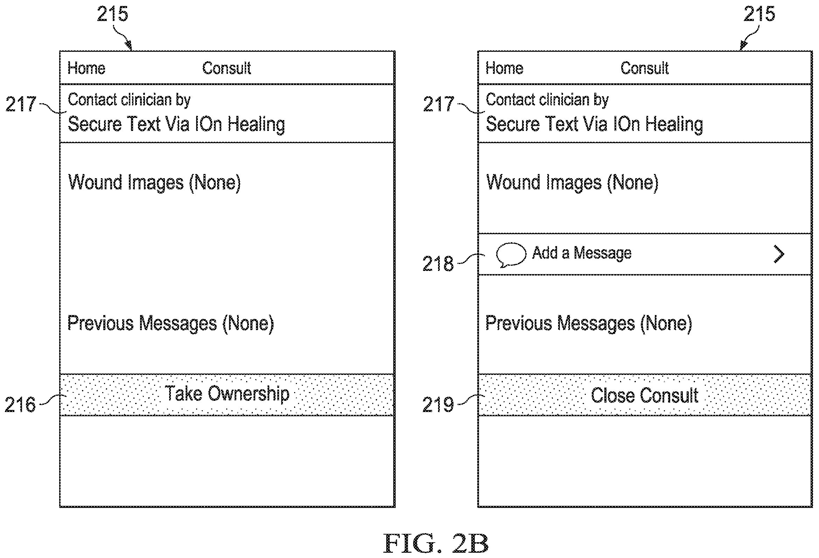

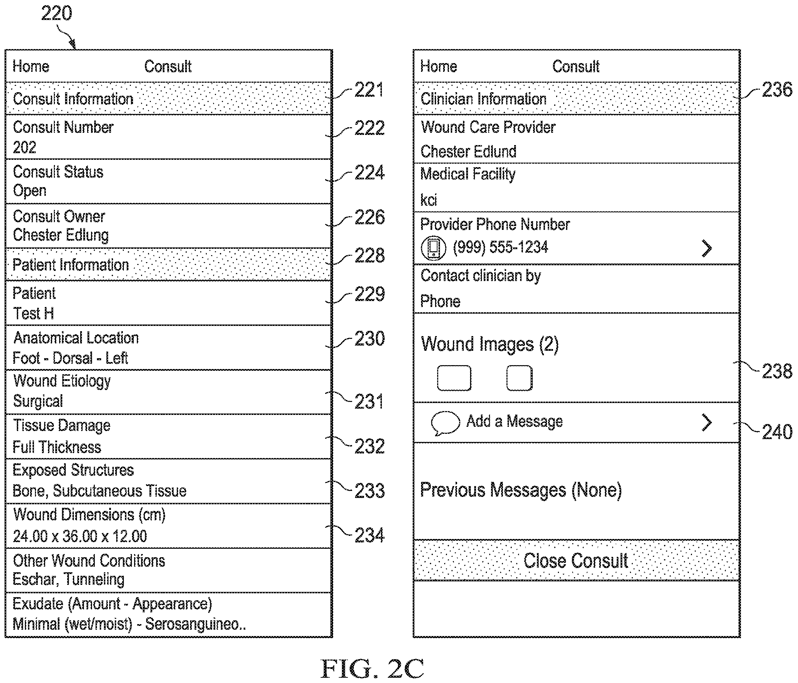

Referring collectively to Figure 2, screen shots of an illustrative graphical user interface (GUI) 200 of an exemplary embodiment of the wound management application are shown. Such wound management application may operate on a mobile device, such as a smartphone. Following logging into the wound management application, a user, such as a regional sales representative, may be presented with a menu bar 201, which in some embodiments may be located across the bottom of the GUI 200. The menu bar 201 of the GUI 200 may include a number of selectable graphical elements, including a "Your Consults" soft-button 202, "New Consults" soft-button 204, "Search" soft-button 206, "Contact Us" soft-button 208, "Sign Out" soft-button 210, along with soft-buttons assigned to any other features related to wound management. The user may select any of these functions (i.e., your consults, new consults, search, contact us, sign out) to cause another GUI for performing the selected function to be presented to the user. For example, the "Your Consults" soft-button 202 may function to display a list of the consults owned by the particular user, while the "New Consults" soft-button 204 may function to display a list of new consults not yet owned by, or assigned to, any user. The "Search" soft-button 206 may allow a user to find any consult - new, owned, or closed. Additionally, the "Contact Us" soft-button 208 may allow a user to send questions, comments, or suggestions about the wound management application to the software development team. The "Sign Out" soft-button 210 may allow a user to securely log out of the wound management application. It should be understood that the GUI 200 is exemplary and that other and/or alternative functions and selection elements may be provided to the user.

For example, the screen shots of Figure 2 provide example illustrations of a consult management functionality of the wound management application. Figure 2A illustrates an example embodiment of the "New Consults" view 212. In this example embodiment, the "New Consults" view 212 includes a patient list 214 in chronological order of new patients that have been treated at a particular wound care center, who may require a consult with a sales representative or a clinical representative. In some embodiments, consultation requests from healthcare providers, such as clinicians, in the geographic territory assigned to a user, such as a regional sales representative, will be sent directly to the user and the user's support team and displayed in the new consults view 212. In other embodiments, these consultation requests may be classified as "not yet owned," and the patient list 214 may be presented simultaneously to multiple users in order to allow for a selective assignment process. In still other embodiments, the wound management application may have automatically already assigned the new consults to one or more specific users, based on criteria processed by the wound management application.

Figure 2B illustrates an example embodiment of a "Review New Consult" view 215, which allows a user, for example a regional sales representative or a clinical representative, to review any preliminary information related to a particular patient, and in some instances, to assign himself or herself to the patient. In some embodiments, the patient may be one of the patients presented on the patient list 214 in the "New Consults" view 212 shown in Figure 2A. Using the "Take Ownership" soft-button 216, the user may alert the wound management application that the patient has now been assigned, and therefore, the patient will subsequently be removed from the patient list 214 on the "New Consults" view 212 that may be displayed simultaneously to multiple users. The patient will then be displayed in the assigned user's list of consults, which may be accessed by the user by selecting the "Your Consults" soft-button 202. Additionally, when a user takes ownership of a consult, a consult status indicator 213 for that respective patient consult may change colors on the patient list 214 for that particular user. For example, a blue dot for the consult status indicator 213 may indicate that a consult is new or that there is some new activity associated with that particular consult, such as a text message or wound images received. Once the user has reviewed the new consult or new material associated with the consult, the consult status indicator 213 may change to no color.

Once the consult is owned by a specific user, the options for communicating with the healthcare provider, for example the clinician, such as texting and calling, may be enabled. For example, the user may select the "Contact clinician by" soft-button 217 and tap the phone number to dial directly, or may select the "Add a Message" soft-button 218 to begin texting. Additionally, once the ownership of the consult has been taken by a user, the "Close Consult" soft-button 219 is activated on the "Review New Consult" view 215 for that respective user. Should the "Close Consult" soft-button 219 be selected by the user, and the consult closed, the consult status indicator may change to red.

To view a particular consult, a user may select that particular consult from the user's list of consults by selecting the "Your Consults" soft-button 202 in the menu bar 201 of the GUI 200. Once selected, the individual consult's information may be displayed on a "Manage the Consult" view 220. In some embodiments, the "Manage the Consult" view 220 may include a "Consult Information" view 221. The "Consult Information" view 221 may display information related to the particular consult. Such consult-related information may include a consult number 222 assigned to the particular consult, a consult status 224 indicating whether the consult is still open or has been closed, and a consult owner 226 field, which in some embodiments may indicate the sales representative assigned to the consult.

Additionally, the "Manage the Consult" view 220 may also include a "Patient Information" view 228. The "Patient Information" view 228 may provide data fields for further descriptors related to one or more wounds of a patient. For example, a patient field 229 may provide some identification information related to the patient. An anatomical location field 230 provides a field for a descriptive location on the body of the patient where the wound is located. A wound etiology field 231 provides a field for entering and displaying the type of wound. For example, the wound etiology field 231 may indicate that the wound is a burn, surgical wound, ulcer, or other type of wound. The "Patient Information" view 228 may also include additional descriptive fields related to the physical nature of the wound, such as a tissue damage field 232 and an exposed structures field 233. The tissue damage field 232 may allow for a description of how many layers of skin tissue are damaged, or in other words, the depth of the wound. The exposed structures field 233 may provide a space for listing any nearby structures at the tissue site that are exposed or otherwise possibly affected by the wound. A wound dimensions field 234 may allow for dimensions of the wound to be entered, including length, width, and depth of the wound. The wound dimensions may be either manually entered into the wound dimensions field 234, or alternatively may be automatically determined by the wound management application based on three-dimensional analysis of the wound images. Additionally, the patient information view 228 may include additional or alternative data fields, based on clinical applications and needs.

The "Manage the Consult" view 220 may further include a "Clinician Information" view 236. The "Clinician Information" view 236 may provide data fields for containing information related to the healthcare provider of the particular consult. For example, fields identifying a particular wound care provider, the medical facility where the patient is being treated, and contact information for the provider may be included. In some embodiments, the "Clinician Information" view 236 may allow a user to tap a phone number included in one or more contact information fields to directly dial the healthcare provider from within the wound management application.

The "Manage the Consult" view 220 may also include a wound images field 238, for displaying images of one or more wounds. The wound images field 238 may allow for a user to tap on a particular image for a full screen view, and may also allow the user to spread and enlarge the image for a more detailed view of particular wound aspects. The wound images displayed in the wound images field 238 may be images taken by any number of individuals, such as one or more health care providers, the consult owner, or even the patient. In some embodiments, the wound images may be transmitted or shared among one or more of the interested parties using the wound management application.

Additionally, some embodiments of the patient monitoring software may include a messaging display 240 as part of the "Manage the Consult" view 220. The messaging display 240 may allow for users to send and receive secure text messages to health care providers as well as other consult owners. Additionally, the message display 240 may include fields for displaying a message log of previous messages that were exchanged between other users of the wound management application, such as previous consult owners for that particular patient.

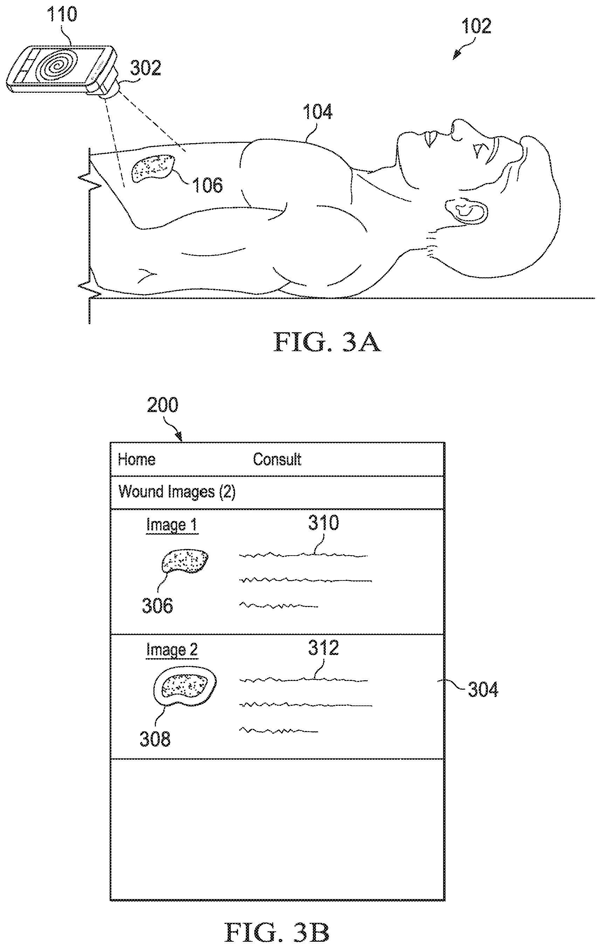

Referring to Figure 3A, an exemplary patient environment, such as clinical setting 102, is shown with the patient 104 having a tissue site 106. Mobile device 110 is also shown, with an image capture device 302, which may be utilized to capture an image of the tissue site 106 in a photograph. The captured image may then be transmitted from the image capture device 302 to the mobile device 110. The image capture device 302 may be a digital camera, mobile telephone, or any other electronic device configured to capture an image in a digital or analog format. In general, to expedite capturing and working with an image of the tissue site 106, the image capture device 302 may be in the form of a digital camera that is configured to be physically connected to the mobile device 110 and may communicate with the mobile device 110 using a wired connection. Alternatively or additionally, the image capture device 302 may be configured to be wirelessly connected to the mobile device 110. In some embodiments, the image capture device 302 may utilize a memory device (not shown) that may be transferred between electronic devices. The memory device may include flash memory, a memory stick, or any other memory device with which the mobile device 110 may be compatible.

As previously discussed, the image capture device 302 may be used to capture images of one or more wounds of a patient, which may then be incorporated into the wound management application. The captured images may then be shared among interested parties such as the clinician, sales representative, clinical representative, and the patient. Wound images captured by the image capture device 302 may be used by the wound management application to determine and subsequently populate one or more wound dimension fields, as discussed with respect to Figure 2. As also previously mentioned, the image capture device 302 may be a three-dimensional camera connected to the mobile device 110, which may also be used to capture wound images that may be used by the wound management application to automatically determine one or more wound dimensions and upload the dimensions to the proper data fields in the wound management application. Additionally, the image capture device 302 may be used to capture images of the tissue site 106 over time, in order for a clinician, sales representative, clinical representative, or other interested party to properly monitor how well the tissue site 106 is healing. Users, such as clinicians, may also have the ability to upload images previously taken, which may be stored in a secure gallery on a mobile device. Tissue site images captured by the image capture device 302 may each be stored in an image database associated with the wound management application and therapy network 100.

Figure 3B shows a screen shot of the illustrative GUI 200 with an example embodiment of a "Wound Images" view 304. In some embodiments, the "Wound Images" view 304 may be accessed through the wound images field 238 of the "Manage the Consult" view 220. The "Wound Images" view 304 may display one or more images, such as Image #1 306 and Image #2 308, of a single tissue site, such as a wound, or of multiple tissue sites for a given patient. The "Wound Images" view 304 may be configured to allow the user to edit an image, enlarge the image for a more detailed view, draw traces around the image of the tissue site, or perform other types of image modification or manipulation. The "Wound Images" view may also include data entry fields, such as notes fields 310 and 312, for allowing a user to enter descriptions, measurements, or other information relevant to the respective image.

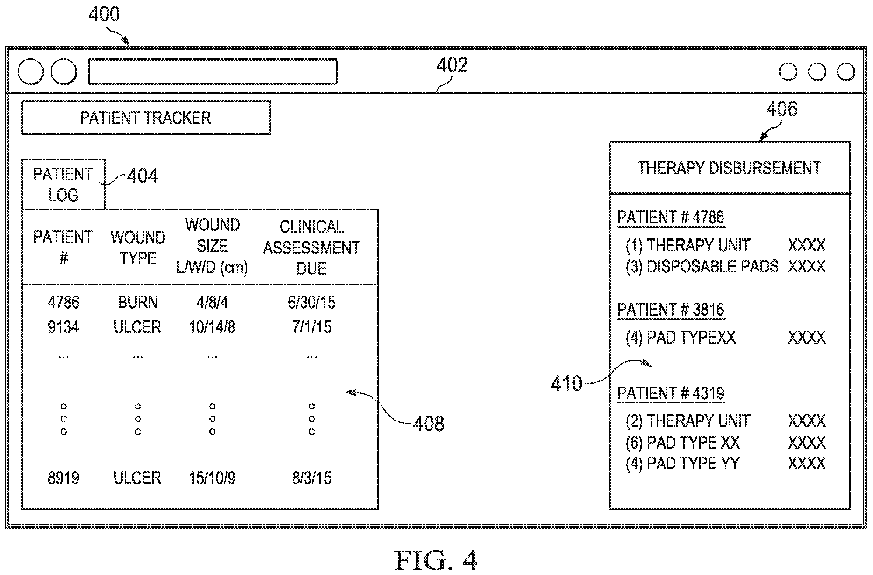

Figure 4 shows a screen shot of an illustrative GUI 400 of an exemplary embodiment of wound management application, or another embodiment of the GUI 200 or a portion of the GUI 200 for use on an electronic device, such as a computer or a mobile device having an electronic display. This exemplary embodiment of GUI 400 provides a user summary view 402, which includes a patient log display 404 and a therapy disbursement display 406.

The patient log display 404 may correspond to patient data 408, such as wound-related data, for a list of one or more patients. The patient log display 404 may display information based on data from the wound management application, which may have been entered by a clinician, sales representative, clinical representative, patient, or other interested party. The data may be interpreted and manipulated by the wound management application in preparation for display. For example, the patient data 408 may display data relating to physical measurements of one or more wounds for a given patient, such as length, width, depth, circumference dimensions, as well as others. Associated with this data, the wound management application may display data corresponding to the healing progress undergone by one or more specific wounds. Additionally, the wound management application may parse wound type, wound size, and other data to calculate potential due dates for when clinical assessments may be required to be included in patient data 408.

The therapy disbursement display 406 may display patient order data 410 related to therapy equipment requested, ordered, and/or disbursed to individual patients. For example, for a given patient, patient order data 410 may report that a specific items associated with a specific therapy regimen were disbursed by the products disbursement center 222. In some embodiments, the patient order data 410 may display, in a list format, the number of each specific therapy unit and/or dressing replacements that have been disbursed on behalf of the patient. The patient order data 410 may also include information related to orders, such as quantities and expected fulfillment dates, for future disbursement that have been placed by a clinician or sales representative for a given patient. The patient order data 410 may further display dates associated with an automatic refill schedule, which may be calculated and provided by the wound management application.

While the illustrative GUI 400 of Figure 4 is focused on information related to individual patients, other illustrative GUIs may include reports focused on summary data for a broad base of patients. For example, other illustrative GUIs may provide a user, such as a regional sales representative, with a list, or alternatively a calendar view, of assigned consults requiring urgent or upcoming attention. Additionally, other illustrative GUIs may provide data related to a summary overview of a given patient population, which may provide for reporting and analytics opportunities. Such overviews may include viewing patient data on a macro level, for example, by grouping patient data by patient demographics, payer groups, wound types, or comorbidities. Providing summary views of one or more patient populations and/or types of wounds may allow for better insight into patient care on a broader scale.

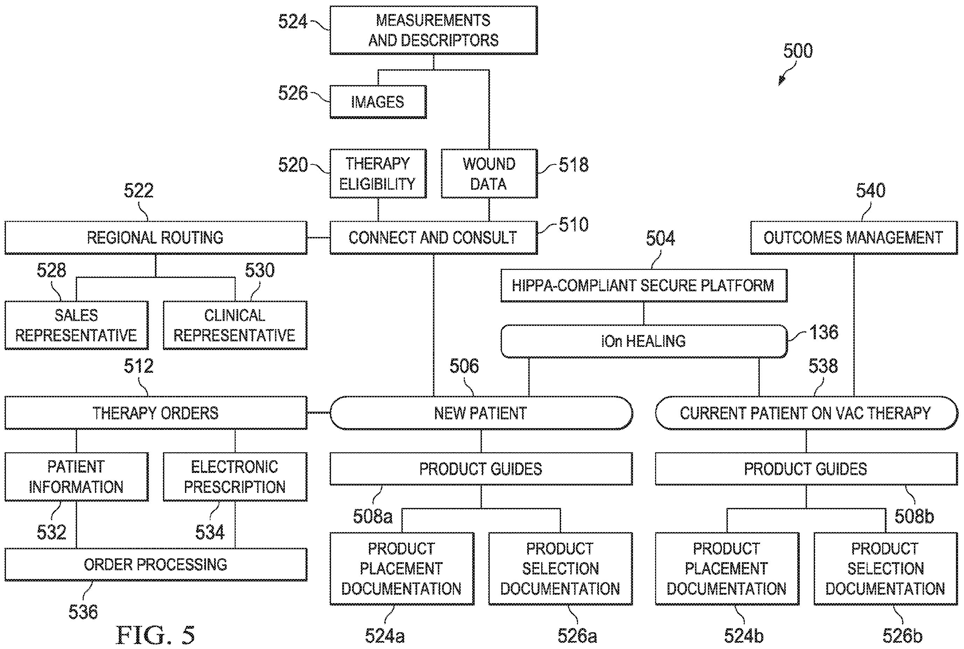

Figure 5 is a functional diagram of an example embodiment illustrating capabilities of a wound management application for use on therapy network 100. As discussed above with respect to Figure 1B, the wound management application may be hosted by a secure server, which may include the iOn Healing Database 136, and may be based on a HIPAA-compliant secure platform 504. The wound management application may be operable on any suitable platform, including iOS, Android, and an HTML-based platform. The wound management application may provide various functions depending on the particular patient and type(s) of wounds presented, as well as the type of user (i.e., clinician, patient, sales representative, etc.). For example, for a new patient 506, the wound management application may provide multiple functional modules, including, but not limited to, product guides 508a, connect and consult 510, and therapy orders 512. The product guides module 508a may provide an interface for a user to read and review product placement documentation 514a and product selection documentation 516a, which may provide product selection recommendations for different wound types and wound locations on a patient.

The connect and consult module 510 may provide an interface for a clinician to create a new wound consult request. In some embodiments, the connect and consult module 510 may include a wound data module 518, a therapy eligibility module 520, and a regional routing module 522. The wound data module 518 may provide an interface for a user, such as a clinician, to collect and assess data for one or more wounds. For example, a clinician may interface with multiple screen shots associated with the measurements and descriptors module 524 to enter dimensions and various other categories of descriptive information for one or more wounds. Closely related, in some embodiments, the wound data module 518 may include an images module 526, which may provide the functional interface for a user, such as a clinician, to capture, edit, and assess images of one or more wounds. As discussed with respect to Figure 2, in some embodiments the images module 526 may be configured to capture and process three-dimensional images of a wound site for automatically determining various wound descriptors.

The therapy eligibility module 520 may be configured to assess and analyze patient data entered by a user, such as a clinician, to determine which, if any, forms of therapy may be applicable to a specific wound site of a patient. For example, the therapy eligibility module 520 may be configured to consider a variety of inputs related to a wound site, and provide a recommendation of whether the patient would be eligible for V.A.C.® Therapy, advanced wound dressings by SYSTAGENIX, or epidermal grafting (CelluTome™), as determined by guidelines by therapy regimen. V.A.C.® Therapy is described in further detail in U.S. Patent Application No. 11/901,657, which is hereby incorporated by reference in its entirety. In some embodiments, the therapy eligibility module 520 may access data gathered and processed by the wound data module 518 as inputs for making the recommendation of applicable forms of therapy, as well as appropriate dressing sizes available to better tailor to the specific needs of the patient.

The regional routing module 522 may be configured to provide a user with the opportunity to request a consult from one or more support representatives. In some embodiments, the regional routing module 522 may include a sales representative module 528, which may allow for the user to request a consult with a specific sales representative. In some embodiments, the sales representative module 528 may be configured to automatically request a consult with either a specific sales representative individual or with a sales representative who matches specific criteria, which may include the location of the clinician. The clinical representative module 530 may provide an interface for selecting a clinical representative, and may have similar functionality as the sales representative module 528. In some embodiments, the clinical representative module 530 may automatically communicate with the wound data module 518 and the therapy eligibility module 520 in order to place a request for a clinical representative consult, should one be warranted. Once the consult request has been routed to the appropriate sales or clinical team member, the team member may take ownership of the consult. Communications between the clinician and sales and/or clinical team members may then occur, by either using a messaging feature integrated within the wound management application or via a phone conversation.

The therapy orders module 512 may provide the integrated ability to place orders for treatment equipment and disposables based on the type of therapy regimen prescribed for a given wound site. The therapy orders module 512 may include a patient information module 532 and electronic prescription module 534. The patient information module 532 may provide the personal information for a patient, which may be used for verifying and processing prescriptions for one or more therapy protocols. The electronic prescription module 534 may provide an interface for a clinician to prescribe therapy regimens, such as V.A.C.® Therapy, directly through the wound management application. The clinician may provide an image of a prescription and attach the image to an order submission or an electronic signature process. In some embodiments, the electronic prescription module 534 may be configured to automatically determine and place orders for the necessary equipment and disposables based on the prescribed therapy regimen. The prescription order may then be processed by the appropriate team to verify patient information and ship-to location(s). The order processing module 536 of the therapy orders module 512 may be configured to communicate with suppliers of therapy products, such as the products disbursement center 122 or various external product vendors. Using the selected product selection documentation 526 and the product placement documentation 524, the appropriate products may be ordered and implemented.

The wound management application may also provide multiple functional modules related to existing patients, for example a current patient on V.A.C.® Therapy 538. Similar to the new patient 506, the wound management application may also include a product guides module 508b for the current patient on V.A.C.® Therapy 538. As discussed above with respect to the product guides module 508a, the product guides module 508b may include the product placement documentation module 514b and the product selection documentation module 516b. Additionally, for current patients, such as the current patient on V.A.C.® Therapy 538, the wound management application may include an outcomes management module 540, which may enable a clinician to submit outcomes management data to other internal or external entities. The outcomes management module 540 may include a variety of capabilities related to tracking wound healing progression data, which may include data related to wound descriptors, wound measurements, wound images, therapy status, and other appropriate clinical notes. For example, in some embodiments, the outcomes management module 540 may communicate with the wound data module 518 in order for comparing beginning wound dimensions and descriptions with periodic measurements and descriptions taken following periods of administered therapy. In some embodiments, the outcomes management module 540 may route outcomes management data for one or more wounds of a patient to an appropriate team based on patient location, which may then submit data to external entities, such as third-party payers, for reimbursement purposes. The outcomes management module 540 may also be configured to communicate securely with external entities, such as providers or even patients themselves, for providing updates to patient medical records related to treatment of one or more wounds.

The wound management application may be further customized to include additional functional modules for offering further capabilities and services to users of the application. For example, one or more functional modules related to detecting leaks of dressings applied to wound sites may be integrated. Additionally, a quality tracking functionality may be included in the wound management application, which may have the ability to track customer complaints, including associated pictures and documentation through a secure interface into customer and/or quality management systems. The wound management application may also include training modules, which may be configured to offer clinicians tutorials on utilizing the application as well as training videos for working with more complex or specialty wound treatment products. Links to clinical guidelines may also be provided through the application in order to provide easy access to reference materials for clinicians.

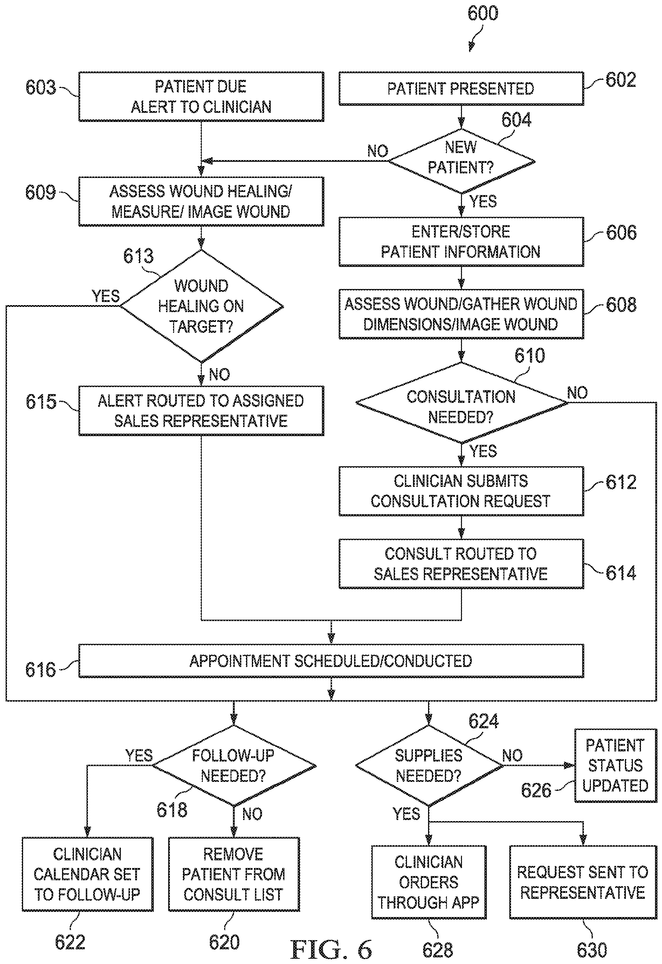

In operation, the therapy network 100 and its various components and features, including the wound management application, may be used in accordance with the exemplary embodiment of a treatment method 600 illustrated in Figure 6. For example, operation of the therapy network 100 and wound management application may begin with a patient being presented to a clinician, as shown in step 602. Alternatively, a clinician may receive a patient due alert 603, which may provide the clinician with a notification that an existing patient is due for a follow-up examination and/or treatment. Following step 602, upon being presented to the clinician, the patient's information may be collected and entered into the wound management application, for example through the use of a mobile device, to determine whether the patient presented is a new patient or a returning patient, as shown in step 604. If the wound management application determines that the patient is a new patient, the clinician or other user may enter and store the new patient's information into the wound management application, according to step 606. The user may make this entry of patient information either directly through a mobile device, such as mobile device 110, or alternatively, may use a computer or other input device. As shown in step 608, the clinician may then assess the patient, which may include making assessments of any wounds presented. For each wound presented, the clinician may gather and enter wound dimensions, which may be linked to the patient's records in the wound management application. Images of any wounds may also be captured, and in some embodiments, an image capture device associated with a mobile device may be used to capture the images, as previously discussed with respect to Figure 3A. Similarly, following a clinician receiving a patient due alert in step 603, or if the wound management application determines that the presented patient is not a new patient in step 604, existing wounds may be assessed to monitor healing and/or to note any potential setbacks or complications, as shown in step 609.

Returning to the scenario where the patient is determined to be a new patient in step 604, once the patient has been examined and any presented wounds have been assessed, the determination of whether a consult with a representative, such as a sales representative or clinical representative, is warranted or required may be made based on the data gathered in step 608, as shown in step 610. In some embodiments, the wound management application may be configured to automatically alert the clinician that a patient consult is required, based on a preliminary analysis of the entered patient data. If it is determined that a consultation is needed with a clinical representative or a sales representative, a consultation request may be submitted, as indicated in step 612. In some embodiments, the clinician may manually make the request for a specific type of consult directly through a mobile device, such as mobile device 110. In other embodiments, the consult request may be automatically made by the wound management application based on data entered regarding the patient. Once the consult request has been initiated, the request may be routed, as shown in step 614, to the appropriate party. The appropriate party for receiving a consult request may be a regional sales representative, a clinical representative, a member of the clinical alert center 120, a member of the products disbursement center 122, or a combination thereof.

In the situation where a returning patient with one or more wounds is being assessed in accordance with step 609, following the assessment of each wound of a patient being monitored, the determination may be made whether healing of the respective wounds is progressing according to an appropriate timeline, as shown in step 613. The clinician may make one or more of these determinations, or alternatively, in some embodiments, the wound management application may make a determination for each wound based on the inputs received for each wound, such as wound dimensions or images, as part of step 609. The wound management application may also allow for the clinician to enter clinical progress reports (CPRs), which may be shared with regional sales and clinical representatives. Should the determination be made that one or more wounds is not healing appropriately, or a further condition or complication is presented, an alert may be routed by the wound management application to an assigned sales or clinical representative, as shown in step 615.

Following either step 614 for a new patient or step 615 for a returning patient, once an alert or consult request is submitted by a clinician or other user, the request is evaluated by the wound management application and routed to an appropriate representative, such as a sales or clinical representative, so that a consult appointment may be scheduled, as shown in step 616. In some embodiments, the request may be sent and/or evaluated by a team member at the sales and clinical support center 116. In some embodiments, the wound management application may automatically route the request for a consult to a particular representative as part of step 616, and in some instances, that representative's consult list may be updated to reflect that a consult is requested. The representative user may use the wound management application to schedule and conduct the requested consult. For example, upon receiving a notification of a consult request, a sales representative may use a mobile device, such as mobile device 110 to communicate with a clinician and/or patient through the wound management application, and more specifically using a GUI such as GUI 200 depicted in Figure 2.

In some situations, it may be determined that an immediate consult is not required following the assessment of a particular wound(s). For example, in step 613, if a particular wound is properly healing, no alert or urgent consult request may be initiated. Similarly, in step 610, following a clinician's evaluation of a new patient's wound(s) according to step 608, the clinician may determine that an immediate consultation with a representative, such as a sales representative or clinical representative is not needed. In either of these scenarios, the user of the wound management application, such as a clinician, may be given one or more prompts by the software, for example through GUI 200, to update the patient's profile. For example, a clinician may be prompted to select whether or not a follow-up consult or appointment is needed at some point in the future, as shown in step 618. In some cases, the clinician may determine that no further appointments are needed, at which point the patient may be removed from the clinician's consult list, according to step 620. For example, in some embodiments the wound management application may automatically remove the patient from the consult list of another user, such as an assigned sales representative or clinical representative. Alternatively, if a clinician makes the assessment that a follow-up consult may be required in the future with a user such as a sales and/or clinical representative, the clinician may make that request directly through the wound management application using his or her mobile device, such as mobile device 110.

Depending on the particular desired configuration of the wound management application, a variety of actions may be taken following the clinician requesting a follow-up consult and/or appointment. For example, in some embodiments, the clinician may be able to schedule an appointment to personally follow-up with the patient, in which case the clinician may be presented with a calendar GUI as part of the wound management application that allows the clinician to reserve an appointment from one or more available appointments, as shown in step 622. In some embodiments, upon a clinician establishing a follow-up appointment, an assigned user, such as a sales representative or clinical representative, may receive an automatic notification through the wound management application of the patient's status. This may provide the user, such as a clinical representative, to review the patient's information and status though one or more GUIs, such as the multiple different views of GUI 200. In some cases, this may provide the clinical representative with an opportunity to send secure messages, such as for offering recommendations for follow-up treatment, to the clinician prior to the patient's next appointment with the clinician. Similarly, a sales representative may also be notified through the wound management application of a patient's scheduled follow-up appointment, and may have the opportunity to recommend therapy options, such as particular wound dressings, directly through a GUI, for example on mobile device 110.

Also following the determination in either step 610 or in 613 that an immediate consult with a sales or clinical representative is not needed, the user, such as the clinician, may be presented by the wound management application through a GUI, such as GUI 200, with the option to select whether the patient is in need of any therapy materials or supplies, as shown in step 624. Should the user, such as a clinician or even the patient himself or herself, indicate through the GUI that no treatment supplies, such as replacement disposable dressing materials, are needed, the patient's current status and profile may be updated by the wound management application in step 626, and an alert may be set for the clinician or other interested party to follow-up with the patient at a particular future date. Alternatively, the user may manually set an alert for a specific date in the future to follow-up with the patient to reevaluate whether any replacement treatment supplies are needed at that time.

Following a user's indication in step 624 that therapy supplies are needed, in some embodiments, the user, such as the clinician, may select from one or more methods of ordering the prescribed therapy units and/or disposables, such as replacement dressing materials. For example, as shown in step 628, the clinician may order therapy units, dressing materials, as well as other disposables for the patient directly through the wound management application, for example through GUI 200 on mobile device 110, and in some embodiments, through an e-script functionality. Alternatively, the clinician may be presented with an option through one or more views in GUI 200 to send a request to a sales representative for a consult or recommendation for specific dressing materials for the given stage of treatment and wound healing progression, as shown in step 630. In response, the sales representative may contact the clinician directly through the wound management application through a secure message to make a recommendation, or alternatively, may directly place an order to the products disbursement center 122 for additional supplies to be disbursed to the clinician or directly to the patient. The sales representative may also send messages through a GUI, such as GUI 200, to either or both of the clinician and patient, for providing specific instructions or advice regarding proper use of the appropriate dressing materials.

The systems and methods described herein may provide significant advantages, some of which have already been mentioned. The wound management application for use on mobile devices provides a HIPAA-compliant tool that can aid in the determination of the applicability of negative-pressure wound therapy or other form of advanced wound care therapy by utilizing the sharing of wound images and standardized wound descriptors to augment verbal descriptions. For example, currently, many clinicians who treat patients with negative-pressure wound therapy do not know which regional sales representative(s) they should contact, or furthermore, how to contact the representative regarding questions they may have regarding therapy options for their patients.

With this wound management application for use on mobile devices, consultation requests from clinicians may be automatically routed to appropriate representatives, such as regional sales representatives and their corresponding assigned team members for expedited response times to the clinician as well as potentially less travel time for sales representatives due to more reliable and standardized wound descriptions. By communicating potentially more accurate and reliable clinical assessment and wound description information, clinicians and sales and clinical representatives may be equipped to provide more appropriate and perhaps better-suited therapy products and regimens. Texting capabilities between clinician and sales and clinical representatives may be performed within their corresponding applications in a secure manner.

The wound management application may also be used for outcomes management purposes to aid in expediting the collection of wound description information that payers often require for reimbursement authorization. For example, current practices involve clinical representatives from treatment supplies providers, such as KCI, calling the clinicians to collect this information or, alternatively, the clinicians are required to fax the needed information to the providers. This wound management application for use on mobile devices can be used by the clinician to directly contact the clinical representative in a HIPAA compliant format using standardized wound descriptions. A calendar within the application reminding the clinician of when each patient is due for wound measurements for outcomes management purposes would also help streamline the reimbursement authorization process.

As previously discussed, the wound management application may also provide improved methods for gathering measurements related to wound dimensions. For example, while current practice involves probing the wound bed to determine wound depth, which can lead to possible pain or discomfort, using a three dimensional camera connected to image the wound can automatically populate wound dimension fields in the application and avoid the need for making physical contact with the wound. Also, based on this wound dimension and/or wound descriptor information inputted by either the clinician or via the three-dimensional camera connected to a mobile device, applicable wound dressings within a product portfolio or specific product line can be recommended. Currently, clinicians often are not fully aware of the extent of the product lines available for treating wound sites. The application, which is accessible through mobile devices, can help inform and guide the clinicians through, not only the different dressing types offered in a product portfolio, such as the KCI wound dressing product lines, but also through the different dressing sizes available to better tailor to the specific needs of a patient. Furthermore, as already discussed, after a recommendation of the appropriate dressing(s) for the patient and/or if the conclusion or consultation results in the determination of negative-pressure wound therapy as an appropriate therapy option, the application can link directly to other processes, such as disbursement functionalities, so that the therapy units and disposable wound dressing components can be ordered directly through the application. An e-script functionality for the prescription of the therapy units may also be included as part of the wound management application. In some embodiments, the wound management application may also provide the functionality for allowing a user, such as a clinician or a patient, to request pickup of therapy units once treatment has concluded.

Although certain illustrative, non-limiting exemplary embodiments have been presented, various changes, substitutions, permutations, and alterations can be made without departing from the scope of the appended claims. Any feature described in connection to any one exemplary embodiment may also be applicable to any other exemplary embodiment. Further, the steps of the methods described herein may be carried out in any suitable order, or simultaneously where appropriate.

Aspects of the invention are disclosed in the following numbered clauses:
1. A processor-implemented method, comprising:
   receiving input information related to a tissue site of a patient;
   providing the input information to a processor for evaluation;
   processing the input information in the processor to identify a selection of actions relating to the tissue site based on the input information;
   generating options in the processor for a user to identify a desired action from the selection of actions;
   providing the desired action identified by the user to the processor; and
   executing the desired action on the processor to provide output information relating to the tissue site.
2. The method according to clause 1, further comprising: providing the output information from the processor to a second user via a communications network relating to the desired action.
3. The method according to clause 1, wherein the input information includes one or more images of the tissue site of the patient provided to the processor.
4. The method according to clause 1, wherein the input information includes descriptive data of a wound at the tissue site.
5. The method according to clause 3, further comprising: determining one or more dimensions of the tissue site from the one or more images.
6. The method according to clause 5, further comprising: populating one or more fields of a graphical user interface (GUI) related to the one or more dimensions of the tissue site.
7. The method according to clause 3, wherein the one or more images of the tissue site are captured by an image capture device in communication with the processor.
8. The method according to clause 7, wherein the image capture device comprises a three-dimensional camera.
9. The method according to clause 6, wherein the one or more dimensions of the tissue site comprises a depth of the tissue site.
10. The method according to clause 1, wherein the options generated in the processor include initiating an electronic message to a support center.
11. A computer-implemented method for treating a tissue site, comprising:
   receiving input information related to a request for a consultation of a tissue site from a first mobile device;
   processing the input information in a processor to identify a selection of consult matches; and
   communicating output information through a communications network to a second mobile device.
12. The method according to clause 11, wherein the step of processing the input information further comprises: calculating geographical data for a group of consult candidates.
13. The method according to clause 11, further comprising: receiving feedback information from the second mobile device related to receiving the request for a consultation.
14. The method according to clause 12, further comprising: updating a consult list for a user of the second mobile device.
15. The method according to clause 13, further comprising:
   receiving therapy recommendation information from the second mobile device;
   processing the therapy recommendation information in the processor to provide output information relating to one or more therapy protocols;
   providing a selection related to the one or more therapy protocols from a user to the processor;
   generating product order information in the processor for communication to one or more therapy product suppliers.
16. A system for treating a tissue site, comprising: a computer processing system comprising a processor adapted to receive input information related to a tissue site and generate output information;
   a mobile device adapted to collect input information related to the tissue site and display output information relating to the tissue site;
   a network adapted to allow communications between the computer processing system and the mobile device.
17. The system of clause 16, further comprising: a database configured for receiving and storing information related to the tissue site.
18. The system of clause 17, wherein the information comprises one or more parameters related to healing of the tissue site.
19. The system of clause 17, wherein the information comprises one or more descriptors related to therapy products.
20. The systems, apparatuses, and methods substantially as described herein.

## Claims

1. A processor-implemented method, comprising:
receiving a plurality of wound images;
upon determining that one of the plurality of wound images is a three-dimensional image, storing wound length information from the three-dimensional image as the wound length information, storing wound width information from the three-dimensional image as the wound width information, and storing wound depth information from the three-dimensional image as the wound depth information;
displaying the wound length information, wound width information, and wound depth information at a wound dimensions field at a Manage the Consult view;
displaying the plurality of wound images in a wound images field at the Manage the Consult View; and
upon a user accessing the wound images field at the Manage the Consult view, displaying the plurality of wound images at a Wound Images view, wherein each of the plurality of wound images is displayed with a data entry field;
wherein the Wound Images view is configured to allow the user to edit the data entry field.

2. The method of claim 1, further comprising the step of receiving wound length information, wound width information, and wound depth information.

3. The method of claim 1, further comprising the step of receiving patient identification information.

4. The method of claim 3, further comprising the step of displaying the patient identification information in a patient field at the Manage the Consult view.

5. The method of claim 1, further comprising the step of receiving anatomical location information.

6. The method of claim 5, further comprising the step of displaying the anatomical location information in an anatomical location field at the Manage the Consult view.

7. The method of claim 1, further comprising the step of receiving wound etiology information.

8. The method of claim 8, further comprising the step of displaying the step of displaying the wound etiology information in a wound etiology field at the Manage the Consult view.

9. The method of claim 1, further comprising the step of receiving tissue damage information.

10. The method of claim 10, further comprising the step of displaying the tissue damage information in a tissue damage field at the Manage the Consult view.

11. The method of claim 1, further comprising the step of receiving nearby structure information.

12. The method of claim 11, further comprising the step of displaying the nearby structures information in an exposed structures field at the Manage the Consult view.

13. The method of claim 1, wherein the Wound Images view is configured to allow the user to edit each of the plurality of wound images.

14. The method of claim 1, further comprising the step of displaying user data at a user summary view, wherein the user data comprises patient identification information, wound length information, wound width information, and wound depth information.

15. The method of claim 1, further comprising the step of recommending a dressing type based on the wound length information, wound width information, and wound depth information.
